Europäisches Patentamt

**European Patent Office**

Office européen des brevets

(11) Veröffentlichungsnummer : **0 133 946**
**B1**

(12) **EUROPÄISCHE PATENTSCHRIFT**

(45) Veröffentlichungstag der Patentschrift :
**28.09.88**

(51) Int. Cl.⁴ : **A 61 B 17/22**

(21) Anmeldenummer : **84108615.0**

(22) Anmeldetag : **20.07.84**

(54) **Einrichtung zum berührungslosen Zertrümmern von Konkrementen.**

(30) Priorität : **03.08.83 DE 3328068**

(43) Veröffentlichungstag der Anmeldung :
**13.03.85 Patentblatt 85/11**

(45) Bekanntmachung des Hinweises auf die Patenterteilung : **28.09.88 Patentblatt 88/39**

(84) Benannte Vertragsstaaten :
**DE FR GB NL**

(56) Entgegenhaltungen :
**DE-A- 2 913 251**

(73) Patentinhaber : **Siemens Aktiengesellschaft Berlin und München**
**Wittelsbacherplatz 2**
**D-8000 München 2 (DE)**

(72) Erfinder : **Pauli, Karlheinz, Dr.**
**Unterer Grenzweg 3**
**D-8524 Neunkirchen (DE)**
Erfinder : **Reichenberger, Helmut, Dr.**
**Begonienstrasse 28**
**D-8501 Eckental (DE)**

## Beschreibung

Die Erfindung betrifft eine Einrichtung zum berührungslosen Zertrümmern eines im Körper eines Lebewesens befindlichen Konkrements mit einem Stoßwellengenerator, der auf ein Zielgebiet ausgerichtet ist.

Einrichtungen dieser Art werden in der Medizin eingesetzt, z. B. zum Zerstören von Steinen in der Niere des Menschen. Sie sind besonders vorteilhaft, da sie jeglichen Eingriff in den Körper vermeiden. Es ist nicht notwendig, operativ vorzugehen. Auch das Heranführen von Sonden und Geräten an das Konkrement entfällt. Eine Gefährdung durch Infektionen oder Verletzungen, z. B. beim Einführen der Sonde oder bei Operationen, kann beim berührungslosen Zertrümmern nicht auftreten.

Eine Einrichtung der eingangs erwähnten Art ist in der DE-AS 23 51 247 beschrieben. Hier wird in einer Fokussierungskammer, die als halbförmiger Rotationsellipsoid ausgebildet ist, in einem ersten Brennpunkt eine Funkenentladung zwischen zwei Elektroden herbeigeführt. Diese verursacht eine Stoßwelle, deren Wellenfront sich nach allen Seiten, d. h. kugelförmig ausbreitet. An der Wand des Rotationsellipsoids werden die Wellen reflektiert. Sie sammeln sich im zweiten Brennpunkt des Rotationsellipsoids. In diesem zweiten Brennpunkt in dem das Konkrement plaziert ist, treffen die reflektierten Wellen gleichzeitig ein. Unter dem gebündelten Anprall der Stoßwellen wird das Konkrement zerstört. Die Ankopplung zwischen der einen Ellipsoidenhälfte und dem Körper, in welchem sich das Konkrement befindet, geschieht über eine dünne Folie, die luftspaltlos am Körper anliegt. Die Fokussierungskammer ist mit Wasser gefüllt.

Diese Einrichtung bringt den Nachteil mit sich, daß Änderungen der Schockwellenenergie nur in geringen Grenzen und nur mit einem erheblichen apparativen Aufwand durch Änderung des Abstands der Unterwasserelektroden möglich sind. Weiterhin ist nachteilig, daß der gegenseitige Abstand der Elektroden zur Erzeugung von intensitätsstarken Schockwellen in der Regel einige Millimeter betragen muß, wodurch die Schockwellenquelle keine punktförmige Geometrie besitzt und Abbildungsfehler bei der Fokussierung entstehen können. Außerdem nutzen sich die Unterwasserelektroden bei jeder Entladung stark ab, so daß ihre Lebensdauer begrenzt ist, was eine regelmäßige Wartung der Einrichtung erforderlich macht.

Obengenannte Umstände sind bereits in der DE-OS 25 38 960 erkannt. Gemäß dieser Druckschrift können die zitierten Nachteile dadurch behoben werden, daß anstelle der Funkenstrecke ein außerhalb der Fokussierungskammer befindlicher Riesenimpulslaser eingesetzt wird. Dessen Strahl wird durch einen Strahlteiler aufgeweitet und dann durch ein in der Wand der Fokussierungskammer befindliches Linsensystem in einem Brennpunkt der rotationselliptischen Fokussierungskammer vereinigt. Hier wird eine Schockwelle ausgelöst, z. B. durch Konzentration des Energiebündels auf einen absorbierenden Stift oder eine stark absorbierende Flüssigkeit. Auch bei dieser Einrichtung wird ein schwierig herzustellender rotationselliptischer Reflexionskörper verwendet. Außerdem ist der Wirkungsgrad einer solchen Einrichtung mit Laser als gering anzusehen. Weiterhin muß angeführt werden, daß die Abstrahlgeometrie ein für alle mal festgelegt ist und das Umgehen vor z. B. Rippen oder anderen Knochen nicht möglich macht, so daß eine Anpassung an die Körperanatomie nur beschränkt möglich ist.

In der DE-OS 29 02 331 ist ein Gerät zur transkutanen, unblutigen Verödung von kleinen retikulären und Besenreiser-Varicen beschrieben. Als Wellengenerator sind gleichzeitig ansteuerbare Ultraschallelemente verwendet, die parabolisch angeordnet sind, damit sich ihre Schallenergien in einem Brennpunkt treffen, in dem die zu zerstörende Varice plaziert ist. Die gesamte Anordnung der Ultraschallelemente ist mittels eines Schneckentriebes und einer Stellschraube längsverschiebbar. Hierdurch können verschiedene Abstände des Brennpunkts vom Applikatorenende eingestellt werden. Bei diesem Gerät ist die Leistung der Ultraschallkristalle zum Zertrümmern von Konkrementen im tiefen Innern eines menschlichen Körpers nicht ausreichend. Die genaue Einstellung der einzelnen Ultraschallschwinger sowohl hinsichtlich des Ortes als auch der Energie ist daher unkritisch. Ein Abschalten einzelner Ultraschallkristalle zwecks Einstellung der Ultraschallenergie im Brennpunkt ist nicht vorgesehen.

In der DE-OS 31 19 295 ist eine Einrichtung zum Zerstören von Konkrementen in Körperhöhlen unter Zuhilfenahme eines großflächigen Ultraschallwandlers als Schwingungserzeuger beschrieben. Es kommt ein fokussierender Ultraschallwandler mit einer Pulsspitzenleistung von wenigstens 100 kW zur Anwendung. Hier besteht die Möglichkeit, verschiedene Zonen des Körpers, die auf dem Schallweg zum Konkrement liegen und dabei stören, durch Verändern der Abstrahlfläche auszublenden. Es wird auch eine Ausführungsform dargestellt, bei der einzelne ringförmige Ultraschallelemente, die den Wandler bilden, auf einer Kugeloberfläche angeordnet sind. Über eine Veränderung der Leistung entsprechend der Art und Tiefenlage des Konkrements ist nichts ausgesagt. Außerdem dürfte der Aufwand für eine solche Einrichtung, insbesondere im Hinblick auf die Ausbildung der ringförmigen Ultraschallelemente, beträchtlich sein.

Außerdem ist anzumerken, daß bei solchen Ultraschallgeräten nur begrenzte Spitzenleistungen der abgestrahlten Schallenergie möglich sind. Darüber hinaus ist bei einem solchen Gerät eine große Anzahl von Ultraschallwandlern erforderlich, was hohen Montage- und Steueraufwand erfordert. Außerdem ist bei Ultraschallwandlern ein unerwünschtes pulsförmiges Unterschwingen zu beobachten, das nur mit erheblichem Aufwand zu reduzieren ist.

In der DE-AS 27 22 252 ist eine Einrichtung zur räumlichen Ortung von Konkrementen beschrieben, wobei die Einrichtung integriert ist in einen Zertrümmerer von Konkrementen. Dieser sogenannte Lithotripter umfaßt ein Koppelgerät in Form eines Ellipsoiden mit einer Stoßwellenquelle im Brennpunkt. An der Wand des Koppelgerätes sind sowohl Ultraschallsender als auch Ultraschallempfänger angeordnet. Die Ultraschallgeräte sind so ausgerichtet, daß sich der gemeinsame Schnittpunkt ihrer abstrahlenden Wellen im körperseitigen, zweiten Brennpunkt des Ellipsoiden überschneiden.

In der DE-OS 29 13 251 ist eine Vorrichtung zur Zertrümmerung von Nierensteinen oder dergleichen beschrieben. Der Stoßwellengenerator ist in einem geschlossenen, mit Wasser gefüllten Gehäuse untergebracht. Ein die Stoßwelle reflektierender Reflektor ist dem Stoßwellengenerator zugeordnet. Die Ankopplung der Stoßwelle an den Körper des Patienten erfolgt über einen verformbaren, nachgiebigen Sack oder Beutel. Der Sack ist mit einem, die Stoßwelle gut leitenden Medium, z. B. Wasser gefüllt. Die beschriebene Vorrichtung umfaßt einen Reflektor, bei welchem Abbildungsfehler nicht unwahrscheinlich sind.

In der Zeitschrift akustische Beihefte, 1962, Heft 1, Seiten 185-202, ist der Aufbau eines sogenannten « Stoßwellenrohres », wie es im Prinzip in der vorliegenden Erfindung verwendet wird, beschrieben. Vor einer Flachspule, durch eine Isolierfolie getrennt, befindet sich eine Kupfermembran. An diese Kupfermembran schließt ein mit Wasser gefülltes Rohr an. Durch Anlegen einer Spannung im Bereich 2-20 kV an die Flachspule wird in der Kupfermembran ein Magnetfeld induziert, welches Abstoßkräfte bewirkt, die die Membran von der Spule schlagartig wegdrücken. Hierdurch entsteht eine Schockwelle, die im wassergefüllten Rohr zu einer steilen Stoßwelle wird und am Rohrende für Experimente zur Verfügung steht. Eingesetzt wird ein solches Stoßwellenrohr z. B. zu Stoffuntersuchungen in der Chemie.

Aufgabe vorliegender Erfindung ist es, bei einer Einrichtung der eingangs genannten Art die Einstellung der Schockwellenenergie variabler zu gestalten. Außerdem sollen Abbildungsfehler bei der Fokussierung möglichst klein gehalten und Wartungsarbeiten an der Einrichtung reduziert werden.

Diese Aufgabe wird erfindungsgemäß dadurch gelöst, daß als Stoßwellengenerator mehrere an sich bekannte, im wesentlichen ebene Stoßwellen erzeugende Stoßwellenrohre vorgesehen sind, die so ausgerichtet sind, daß sich die Mittelachsen der von ihnen ausgehenden Stoßwellen im Zielgebiet in einem Schnittpunkt schneiden.

Da mehrere Stoßwellenrohre vorgesehen sind, erlaubt diese Einrichtung durch Zu- oder Abschalten einzelner Stoßwellenrohre nach Bedarf eine stufenweise Einstellung der im Zielgebiet wirkenden Stoßwellenenergie. Diese wählbare Einstellung ist besonders wünschenswert, da sich die einzustrahlende Energiemenge nach Art, Größe, Gestaltung, chemischer Zusammensetzung, etc. des zu zertrümmernden Objekts richtet. Außerdem besitzen die Stoßwellenrohre selbst keine Funkenstrecke ; vielmehr verursachen sie den Stoßimpuls durch magnetische Kräfte. Dadurch werden Wartungsarbeiten, die bei einer Ausführungsform nach dem Stande der Technik durch Elektrodenabbrand hervorgerufen werden, vermieden. Außerdem ist die zeitliche und räumliche Reproduzierbarkeit der Stoßwelle durch Vermeidung einer Funkenstrecke zu deren Erzeugung sehr hoch. Darüber hinaus bietet eine Stoßwellenrohr-Anordnung ein hohes Maß an elektrischer Sicherheit für Patient und Bedienungspersonal.

Da die Stoßwellen nicht über den Umweg der Reflexion in das Zielgebiet gelangen, können daraus resultierende Unwägbarkeiten auch nicht auftreten.

Ein weiterer Vorteil ist, daß, für den Fall, daß nicht alle vorhandenen Stoßwellenrohre gleichzeitig eingesetzt werden, eine Auswahl der nicht teilnehmenden Stoßwellenrohre so vorgenommen werden kann, daß diejenigen, in deren Durchschallungsweg Hindernisse wie z. B. Knochen liegen, abgeschaltet werden.

Weitere Vorteile und Einzelheiten der Erfindung ergeben sich aus der nachfolgenden Beschreibung von Ausführungsbeispielen anhand der Zeichnungen in Verbindung mit den Unteransprüchen.

Es zeigen :

Fig. 1 einen Aufriß einer Zertrümmerungseinrichtung nach der Erfindung mit acht Stoßwellenrohren und mit einem Ultraschallortungssystem,

Fig. 2 einen Schnitt längs der Linie II-II durch die Einrichtung nach Figur 1,

Fig. 3 einen Schnitt längs der Linie III-III durch die Einrichtung nach Figur 1,

Fig. 4 ein Stoßwellenrohr nach dem Stand der Technik,

Fig. 5 einen Längsschnitt durch ein Stoßwellenrohr, dem eine Linse beigeordnet ist, in stark vergrößerter Darstellung gegenüber dem beschallten Organ,

Fig. 6 einen Längsschnitt durch mehrere Stoßwellenrohre, denen eine gemeinsame Linse beigeordnet ist, und

Fig. 7 einen Schnitt entsprechend Figur 3 mit einem stehenden Patienten und mit einer Stoßwellen-Auskoppeleinrichtung.

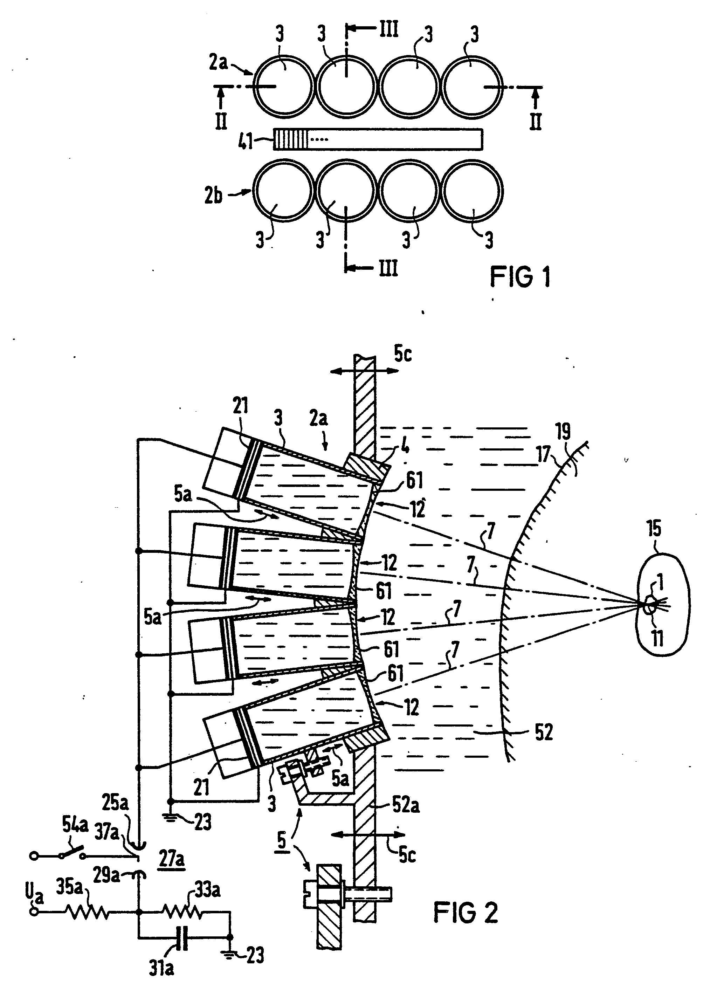

Figuren 1 bis 3 zeigen den Ausschnitt einer Einrichtung zum berührungslosen Zertrümmern eines im Körper eines Lebewesens befindlichen Konkrements 1. Die Einrichtung umfaßt zwei bogenförmige Zeilen 2a und 2b zu je vier an sich bekannten Stoßwellenrohren 3. Die zwei Zeilen 2a, 2b liegen auf einer Kugeloberfläche, und die Stoßwellenrohre 3 sind nebeneinander plaziert, wozu ein Halterahmen 4 dienen kann. Sie liegen jedoch nicht zu dicht, so daß noch eine mechanische Feinregulierungseinrichtung 5, z. B. mehrere Führungen mit feingängiger Spindel, bequem untergebracht und bedient werden können. Bei der Anordnung mehrerer identischer, zylindrischer Stoßwellenrohre 3 sollte darauf geachtet werden, daß die Verlängerungen ihrer Zentrumsachsen 7 sich in einem gemeinsamen Schnittpunkt 11 treffen und daß die Öffnungen der Stoßwellenrohre 3 gleichen Abstand von diesem Schnittpunkt 11 besitzen. Die Austrittsöffnungen 12 der Stoßwellenrohre 3 liegen damit auf einem Kreis um den Schnittpunkt 11. Die Mittelachsen der von den Stoßwellenrohren ausgehenden Stoßwellen verlaufen entlang den Zentrumsachsen 7. Je nach Ausführungsform treffen die Stoßwellen im Schnittpunkt 11 zeitgleich, was für manche Anwendungsfälle bevorzugt ist, oder aber zeitlich gestaffelt oder versetzt ein. Letztere Ausführungsform kann von Vorteil sein, wenn das Konkrement 1 insgesamt gesehen einer längeren Einwirkungsdauer ausgesetzt werden soll.

Die Feinregulierungseinrichtung 5 umfaßt die Möglichkeit, jedes Stoßwellenrohr 3 einzeln in Richtung der Zentrums- oder Längsachse 7 zu verschieben. Dadurch verändert sich der Abstand zwischen der Stoßwellenrohröffnung 12 und dem gemeinsamen Schnittpunkt 11 der einzelnen Längsachsen 7. Dies ist durch einen Pfeil 5a angedeutet.

Zur Einstellung der Tiefe des Schnittpunkts 11 im Körper des Patienten 19 ermöglicht die Feinregulierung 5 außerdem ein Verschieben des Halterahmens 4 mit allen auf ihm montierten Stoßwellenrohren 3. Ein Doppelpfeil 5c deutet dies an.

In dem genannten Schnittpunkt 11 ist das Konkrement 1 angeordnet. Im gezeigten Fall handelt es sich um einen Stein in einer Niere 15. Eine ausgezogene Linie 17 symbolisiert die Körperoberfläche des Patienten 19, der vor der Zertrümmerungseinrichtung plaziert ist.

Jedes Stoßwellenrohr 3 umfaßt eine Flachspule 21, die mit einem Anschluß an gemeinsame Masse 23 gelegt ist. Der andere Anschluß der Flachspule 21 ist, zusammengefaßt mit den entsprechenden Anschlüssen anderer Flachspulen 21 desselben Bogens 2a oder 2b, an eine erste Elektrode 25a bzw. 25b einer geeigneten Schaltvorrichtung, wie z. B. einer Funkenstrecke 27a bzw. 27b oder einem Vakuum- oder SF6-Schalter angeschlossen. Eine zweite Elektrode 29a bzw. 29b der Funkenstrecke 27a bzw. 27b liegt an einem Kondensator 31a, 31b an. Parallel zum Kondensator 31a, 31b liegt ein erster Widerstand 33a bzw. 33b. An dieser Parallelschaltung liegt über einen zweiten, in Reihe geschalteten Widerstand 35a, 35b eine Ladespannung $U_a$ bzw. $U_b$ an. Zwischen den beiden Elektroden 25a, 25b sowie 29a, 29b der Funkenstrecke 27a bzw. 27b liegt eine Steuerelektrode 37a bzw. 37b, mit deren Hilfe die betreffende Funkenstrecke 27a, 27b gezündet wird.

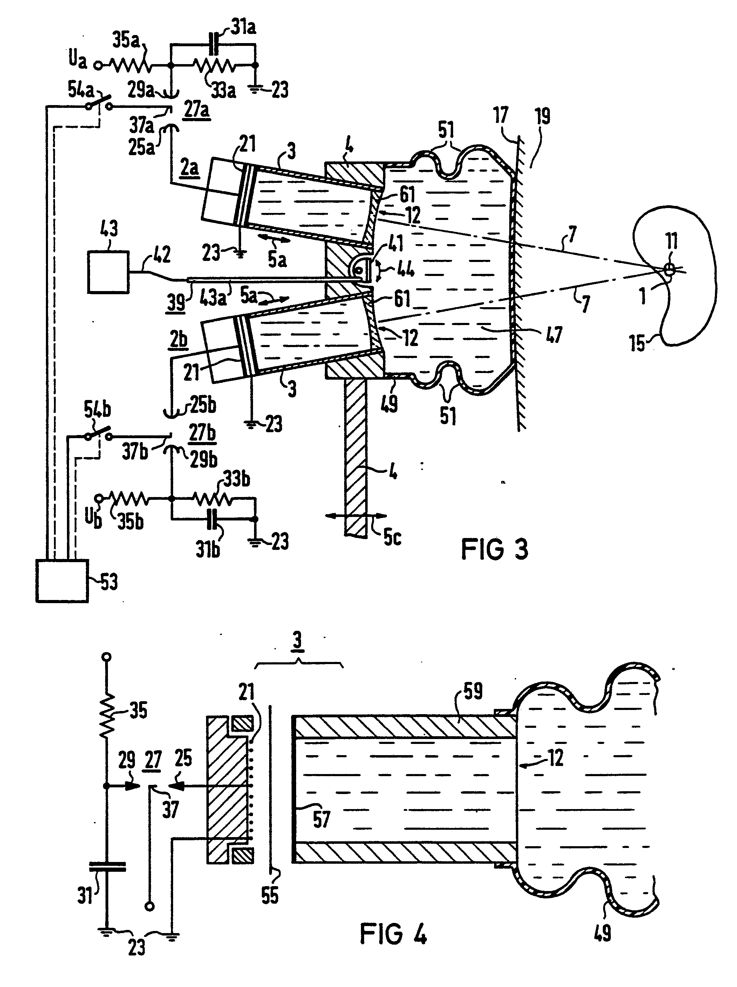

Zwischen den beiden parallelen Zeilen 2a, 2b von Stoßwellenrohren 3 befindet sich eine Ortungseinrichtung 39, welche zum Empfang und zum Senden von Ultraschallsignalen geeignet ist. Diese dient zum berührungslosen Auffinden der Position des Konkrements 1. Sie ist im vorliegenden Beispiel eine Ultraschall-Lokalisierungseinrichtung. Die Ortungseinrichtung 39 umfaßt eine Parallelanordnung 41 von Ultraschallelementen (vgl. Figuren 1 und 3), die über eine Signalleitung 42 mit einer Sende- und/oder Empfangseinrichtung 43 verbunden ist. Die Anordnung 41 ist schwenkbar an einem Stativ mit einer Haltestange 43a gelagert, durch welche gleichzeitig die Signalleitungen 42 von und zu den Ultraschallwandlerelementen geführt sein können. Dadurch ist die Anordnung 41 um ihre Längsachse drehbar, was durch einen Doppelpfeil 44 gezeigt ist. Eine alternative Ausführungsform zu dieser Anordnung 41 der Ultraschallelemente besteht darin, daß die Ultraschallelemente direkt jedem einzelnen Stoßwellenrohr 3 zugeordnet sind. Eine weitere Möglichkeit besteht darin, die Ultraschallwandlerelemente als Flächenarray auszubilden.

Mit Hilfe der Ortungseinrichtung 39 wird der Patient 19 so plaziert, daß sich das zu zerstörende Konkrement 1 im Schnittpunkt 11 der Zentralachsen 7 der Stoßwellenrohre 3 befindet. Die Ankopplung zwischen Stoßwellenrohröffnung 12 und Patient 19 wird über einen mit einem Koppelmedium z. B. Wasser 47 gefüllten, flexiblen Sacke 49 (vgl. Figur 3) hergestellt. Dieser kann insbesondere aus einem elastischen Material mit einer akustischen Impedanz, die der des Wassers ähnlich ist, bestehen. Bevorzugt kommt EPDM-Gummi in Frage. Der Sack 49 ist vorzugsweise am Umfang des Stoßwellenrohrs 3 befestigt. Es ist darauf zu achten, daß der Sack 49 ohne Luftspalt am Körper des Patienten 19 anliegt. Das Anliegen kann durch akustische Kontrollen mit Hilfe der Ultraschalleinrichtung auf Richtigkeit kontrolliert werden. Um den Justieraufwand an den Stoßwellenrohren 3 möglichst gering zu halten, ist es zweckmäßig, die Wand des Sacks 49 so auszubilden, daß an den Seiten Falten 51 vorhanden sind. Diese Falten 51 haben eine balgähnliche Funktion und ermöglichen, die Stoßwellenrohre 3 näher oder weiter entfernt vom Patienten 19 zu plazieren sowie Kippbewegungen in sämtliche Richtungen aufzufangen. Damit ändert sich entsprechend die Lage des Schnittpunkts 11 im Patienten 19. Eine individuelle Justierungsänderung der Stoßwellenrohre 3 selbst ist nicht nötig. Dieser Vorteil wird als besonders bedeutsam angesehen.

Die Ankopplung kann auch über ein Wasserbad 52 in einer Wanne erfolgen, in welchem sich der Patient 19 befindet. Die Stoßwellenrohranordnung ist dabei in einer Aussparung in der Wand 52a der Wanne eingesetzt (Figur 2).

Befindet sich das Konkrement 1 im besagten Schnittpunkt 11, können die Stoßwellen gleichzeitig ausgelöst werden. Die Anzahl der zugeschalteten Stoßwellenrohre 3 hängt von der Größe und Konsistenz des Konkrementes 1 sowie der durch die Anatomie des Patienten vorgegebenen Geometrie ab und ist entsprechend eingestellt. Die einzelnen Stoßwellen durchlaufen auf getrennten Wegen den Wassersack 49 und den Körper bis zum Schnittpunkt 11. Dort treffen sie gleichzeitig zusammen und geben einen Teil ihrer Energie an das Konkrement 1 durch Druck- und Zugeffekt ab. Damit wird dieses zerstört oder zumindest beschädigt. Über die Empfangsmöglichkeit der Ortungseinrichtung 39 für das Konkrement 1 kann aufgrund der reflektierten Schockwelle ein Erfolg des Zertrümmerungsvorgangs kontrolliert werden.

Es sind Anwendungsfälle denkbar, bei denen man die Stoßwellen den vorgegebenen Volumenbereich in dem das Konkrement 1 liegt, in einer einstellbaren zeitlichen Folge durchlaufen läßt. Die Einstellung der zeitlichen Verschiebung ist über die Feinregulierungseinrichtung 5 durch Verändern des Abstands entlang dem Doppelpfeil 5a möglich. Eine andere Variante hierzu ist die zeitlich verzögerte Auslösung der einzelnen Stoßwellen. Figur 3 zeigt hierzu ein Ausführungsbeispiel, bei welchem die Steuerelektroden 37a, 37b voneinander getrennt mit einer Steuereinheit 53 verbunden sind. Die Steuereinheit 53 gibt in der gewünschten Reihenfolge Spannungsimpulse auf die Steuerelektroden 37a und 37b der einzelnen Stoßwellenrohre 3 der Reihen 2a bzw. 2b und bewirkt dadurch die Zündvorgänge. Die Einstellung des Abstands zwischen dem Konkrement 1 und der Stoßwellenrohröffnung 12 erfolgt hier also nicht über die mechanische Feinregulriereinrichtung 5, sondern durch zeitlich versetztes Auslösen der einzelnen Stoßwellen. Hierdurch kann die Anordnung der Stoßwellenrohre 3 auf einem Kreisbogen entfallen. Das Ausrichten der Stoßwellenrohre 3 auf das Konkrement 1 muß allerdings nach wie vor.gegeben sein.

Die zeitliche Versetzung kann z. B. dadurch realisiert sein, daß jedem Stoßwellenrohr 3 ein eigener Schaltkreis mit einer eigenen Schalteinrichtung 54 und Steuerbauelementen zugeordnet ist, die über die gemeinsame Steuereinheit 53 betrieben werden. Die Steuereinheit 53 ermöglicht außerdem die Einstellung von Amplitude und Impulsform der einzelnen Stoßwellen. Dadurch wird weiterhin ermöglicht, die Ortung des Konkrements 1 direkt mit Hilfe des Stoßwellenrohrs 3 vorzunehmen, indem die Amplitude der Stoßwelle entsprechend klein eingestellt wird. In Figur 3 sind hierfür zwei Schalteinrichtungen 54a, 54b eingezeichnet.

Besonders vorteilhaft bei der vorliegenden Erfindung ist, daß in der Regel nicht immer alle Stoßwellenrohre 3 zugeschaltet sein müssen. Dies ermöglicht eine Auswahl der teilnehmenden Stoßwellenrohre 3 so, daß nur diejenigen mit optimalem Strahlungsweg bevorzugt werden. Stoßwellenrohre 3, bei denen Hindernisse, wie z. B. Knochen, in der Stoßwellenlaufstrecke zu beobachten sind, werden bei der Stoßwellenabgabe nicht zugeschaltet.

Figur 4 zeigt einen Längsschnitt durch ein typisches Ausführungsbeispiel eines Stoßwellenrohrs 3. Der Kondensator 31 liegt mit einem Anschluß an Masse 23, sein anderer Anschluß ist an die zweite Elektrode 29 der Funkenstrecke 27 angeschlossen. Die erste Elektrode 25 der Funkenstrecke 27 ist mit dem Anfang der Flachspule 21 verbunden. Das Ende dieser Flachspule 21 ist auf Masse 23 gelegt. Vor der Flachspule 21 ist eine Isolierfolie 55 angeordnet, welche eine Kupfermembran 57 von der Flachspule 21 elektrisch isoliert. Die Kupfermembran 57 ist randseitig mit einem mit Flüssigkeit, z. B. Wasser gefüllten zylindrischen Rohrstück 59 verbunden. Das Ende des Rohrstücks 59 ist als Stoßwellenrohröffnung 12 bezeichnet. Hier kann direkt der Wassersack 49 anschließen.

Zwischen der ersten und zweiten Elektrode 25 bzw. 29 der Funkenstrecke 27 befindet sich die Steuerelektrode 37. Wird auf diese Steuerelektrode 37 ein Spannungsimpuls gegeben, so wird die Funkenstrecke 27 gezündet und somit leitend. Die im Kondensator 31 gespeicherte Energie entlädt sich in die Flachspule 21, welche damit sehr schnell ein magnetisches Feld aufbaut. In der Kupfermembran 57 wird ein Gegenfeld induziert, welches bewirkt, daß die Kupfermembran 57 von der Flachspule 21 abgestoßen wird. Dieses Ausschlagen der Kupfermembran 57 erzeugt eine Druckwelle im Wasser. Die Druckwelle setzt sich fort bis zur Stoßwellenrohröffnung 12, wobei im Verlauf durch das Rohrstück 59 und danach die Wellenfront an Steilheit gewinnt.

Bei geeigneter Dimensionierung der Komponenten der Stoßwellenrohre 3 ist die Druckamplitude der Stoßwellen im Zielgebiet 1 großgenug, um den gewünschten Effekt der Konkrementzertrümmerung zu erzielen.

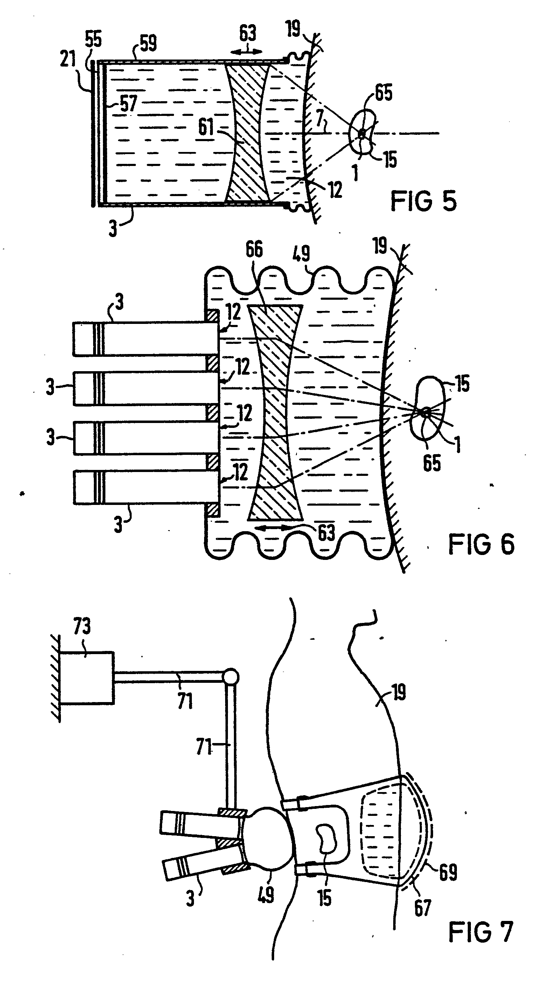

Figur 5 zeigt einen Schnitt längs durch ein Stoßwellenrohr 3 gemäß Figur 4, an dessen Öffnung 12 eine akustische Sammellinse 61 montiert ist. Die Sammellinse 61 kann in Richtung des Doppelpfeils 63 also entlang der Zentrums- oder Längsachse 7 des Stoßwellenrohrs 3 verschiebbar sein. Bei dieser verschiebbaren Anordnung der Sammellinse 61 kann dann auf eine Feinregulierung des gesamten Stoßwellenrohrs 3 gemäß Doppelpfeil 5a in Figur 1 verzichtet werden. Durch das Verschieben der Sammellinse 61 werden auch entsprechend ihre Brennpunkte 65 verschoben, deren Abstand von der Stoßwellenrohröffnung 12 sich somit variieren läßt. In dem körperseitig liegenden Brennpunkt 65 der Sammellinse 61 befindet sich das zu zertrümmernde Konkrement 1. Diese Anordnung der Sammellinse 61 wird zweckmäßigerweise bei allen Stoßwellenrohren 3 vorgenommen. Alternativ hierzu besteht die Möglichkeit, daß sich mehrere oder alle Stoßwellenrohre 3 vor einer gemeinsamen Sammellinse 66 befinden. Eine Anordnung hierzu zeigt Figur 6. Zwischen vier parallel verlaufenden Stoßwellenrohren 3 und dem Körper des Patienten 19 befindet sich eine einzige Sammellinse 66. Deren Durchmesser ist ausreichend groß, um alle ausgesendeten ebenen Wellen zu erfassen. Bezüglich der Lage des Brennpunktes 65 der Linse 66 und dessen Verschiebbarkeit gilt das gleiche wie für die Anordnung gemäß Figur 5. Vorteil dieser Lösung ist, daß nicht jedes Stoßwellenrohr 3 individuell ausgerichtet zu werden braucht, sondern lediglich eine Parallelstellung aller Stoßwellenrohre 3 nötig ist. Die Gesamtanordnung hat nur einen Brennpunkt 65, welcher deckungsgleich zum Konkrement 1 justiert wird.

Figur 7 zeigt eine Gesamtdarstellung der Zertrümmerungseinrichtung mit einer Auskoppelvorrichtung für die Stoßwellen. Der für den Ein- und Austritt der Stoßwellen in Frage kommende Bereich des Patienten 19 ist mit einem herumlegbaren Kunststoffsack 67 nach Art einer Bauchbinde abgedeckt. Die Bauchbinde 67 liegt luftspaltlos am Körper des Patienten 19 an und ist mit einer Füllung, z. B. mit Wasser versehen, die die Stoßwellen gut leitet. An der dem Körper abgewandten Seite werden die Stoßwellen von einem flächenhaften Ultraschallempfänger 69 für Transmission aufgefangen. Die Stoßwellenrohre 3 sind an einem gewinkelten Haltearmgestell 71 befestigt, der allseitige Schwenkungen gestattet. Dieses ist vorteilhaft, um bei der Zerstörung ausgedehnter Konkremente oder bei Patientenbewegungen während der Behandlung den gemeinsamen Schnittpunkt 11 nachzuführen. Das Haltearmgestell 71 kann dabei über eine Befestigungseinrichtung 73 fest mit einer Wand des Behandlungsraums verbunden sein. Zu diesem Haltearm 71 können beispielsweise auch die Teile der Feinregulierung 5 gehören, welche die Verschiebung entlang dem Doppelpfeil 5c gestattet.

**Patentansprüche**

1. Einrichtung zum berührungslosen Zertrümmern eines im Körper eines Lebewesens befindlichen Konkrements mit einem Stoßwellengenerator, der auf ein Zielgebiet ausgerichtet ist, dadurch gekennzeichnet, daß als Stoßwellengenerator mehrere an sich bekannte, im wesentlichen ebene Stoßwellen erzeugende Stoßwellenrohre (3) vorgesehen sind, die so ausgerichtet sind, daß sich die Mittelachsen (7) der von ihnen ausgehenden Stoßwellen im Zielgebiet (I) in einem Schnittpunkt (11 ; 65) schneiden.

2. Zertrümmerungseinrichtung nach Anspruch 1, dadurch gekennzeichnet, daß zum Ausrichten der Stoßwellen auf das Zielgebiet (1) eine mechanische Einrichtung (5, 71) vorgesehen ist.

3. Zertrümmerungseinrichtung nach Anspruch 2, dadurch gekennzeichnet, daß die mechanische Einrichtung Feintriebe (5) umfaßt, die einzelnen Stoßwellenrohren (3) zugeordnet sind und die jeweils zum Einstellen des Abstandes zwischen dem betreffenden Stoßwellenrohr (3) und dem Zielgerät (1) vorgesehen sind (Figuren 1 bis 3).

4. Zertrümmerungseinrichtung nach Anspruch 2 oder 3, dadurch gekennzeichnet, daß die mechanische Einrichtung ein Haltearmgestell (71) umfaßt, das zum Einstellen der Lage des Schnittpunktes (11) aller Mittelachsen (7) dient (Figur 7).

5. Zertrümmerungseinrichtung nach einem der Ansprüche 1 bis 4, dadurch gekennzeichnet, daß die Stoßwellenrohre (3) so angeordnet sind, daß sich ihre Zentrumsachsen (7) in einem gemeinsamen Schnittpunkt (11) schneiden und daß die Austrittsöffnungen (12) der Stoßwellenrohre (3) alle denselben Abstand zu diesem Schnittpunkt (11) besitzen.

6. Zertrümmerungseinrichtung nach einem der Ansprüche 1 bis 5, dadurch gekennzeichnet, daß zur zeitlich variablen Ankunft im Zielgebiet (1) der Stoßwellen eine Einrichtung vorgesehen ist, die die Stoßwellenrohre (3) elektrisch so ansteuert, daß sich eine zeitliche Versetzung der ausgesandten Stoßwellen ergibt.

7. Zertrümmerungseinrichtung nach einem der Ansprüche 1 bis 6, dadurch gekennzeichnet, daß ihr eine Steuereinheit (53) zugeordnet ist, die so ausgebildet ist, daß die Amplitude und/oder die Impulsform der einzelnen Stoßwellen der Stoßwellenrohre (3) einstellbar ist.

8. Zertrümmerungseinrichtung nach einem der Ansprüche 1 bis 7, dadurch gekennzeichnet, daß eine auf dem Linsenprinzip beruhende akustische Fokussierungseinrichtung (61) vorgesehen ist, die die Stoßwellen der einzelnen Stoßwellenrohre (3) jeweils auf das Zielgebiet (1) fokussiert (Figuren 2 und 3).

9. Einrichtung zum berührungslosen Zertrümmern eines im Körper eines Lebewesens befindlichen Konkrements mit einem Stoßwellengenerator, der auf ein Zielgebiet ausgerichtet ist, dadurch gekennzeichnet, daß als Stoßwellengenerator mehrere an sich bekannte, im wesentlichen ebene Stoßwellen erzeugende Stoßwellenrohre (3) vorgesehen sind, die parallel ausgerichtet sind und eine gemeinsame auf dem Linsenprinzip beruhende akustische Fokussierungseinrichtung (66) aufweisen, die die Stoßwellen der einzelnen Stoßwellengeneratoren (3) auf das Zielgebiet (1) fokussiert (Figur 6).

10. Zertrümmerungseinrichtung nach Anspruch 8 oder 9, dadurch gekennzeichnet, daß Fokussierungseinrichtung eine akustische Sammellinse (61 ; 62) umfaßt.

11. Zertrümmerungseinrichtung nach einem der Ansprüche 1 bis 10, dadurch gekennzeichnet, daß die Stoßwellenrohre (3) mittels eines Kopplungsmediums (47) an das Lebewesen (19) ankoppelbar sind.

12. Zertrümmerungseinrichtung nach einem der Ansprüche 1 bis 11, dadurch gekennzeichnet, daß die Stoßwellenrohre (3) über einen mit einen Kopplungsmedium (47) gefüllten ersten flexiblen Kunststoffsack (49) an das Lebewesen (19) ankoppelbar sind, wobei das Kopplungsmedium (47) in seinen akustischen Eigenschaften denen des Wassers ähnlich ist.

13. Zertrümmerungseinrichtung nach Anspruch 12, dadurch gekennzeichnet, daß der erste Kunststoffsack (49) mit Falten (51) versehen ist, die eine balgähnliche Bewegung der Sackwand ermöglichen.

14. Zertrümmerungseinrichtung nach einem der Ansprüche 1 bis 13, dadurch gekennzeichnet, daß die Stoßwellen nach Durchlaufen des Zielgebietes (1) über einen mit einem Ankoppelmedium gefüllten zweiten Kunststoffsack aus dem Körper auskoppelbar sind.

15. Zertrümmerungseinrichtung nach Anspruch 12, 13 oder 14, dadurch gekennzeichnet, daß der erste und zweite Kunststoffsack (49) in einem um das Zielgebiet (1) herumlegbaren dritten Kunststoffsack (67) vereinigt sind.

16. Zertrümmerungseinrichtung nach einem der Ansprüche 1 bis 5, dadurch gekennzeichnet, daß ihr zwecks Ortung des Konkrements eine Einrichtung (41) zum Empfang und zur Verarbeitung von transmittierten und/oder reflektierten akustischen Signalen zugeordnet ist, die durch Wechselwirkung eines ausgesandten Druckimpulses mit dem Körper des Lebewesens (19) und/oder mit dem Koppelmedium entstehen.

17. Zertrümmerungseinrichtung nach Anspruch 16, dadurch gekennzeichnet, daß ihr eine Einrichtung (41) zum Senden des im Ultraschallbereich liegenden Druckimpulses zugeordnet ist, die in die Empfangs- und Verarbeitungseinrichtung (41) integriert ist.

18. Zertrümmerungseinrichtung nach Anspruch 16, dadurch gekennzeichnet, daß als Sender für den im Ultraschallbereich liegenden Druckimpuls zumindest eins der Stoßwellenrohre (3) vorgesehen ist.

19. Zertrümmerungseinrichtung nach einem der Ansprüche 16 bis 18, dadurch gekennzeichnet, daß die Einrichtung zum Empfang ein Ultraschall-Array umfaßt, das um seine Längsachse drehverstellbar ist.

20. Zertrümmerungseinrichtung nach einem der Ansprüche 16 bis 19, dadurch gekennzeichnet, daß die Empfangseinrichtung (41) zwischen den auf das Zielgebiet (1) ausgerichteten Stoßwellenrohren (3) angeordnet ist.

21. Zertrümmerungseinrichtung nach einem der Ansprüche 16 bis 18, dadurch gekennzeichnet, daß die Einrichtung (41) zum Empfang ein Flächen-Array für Transmission umfaßt.

22. Zertrümmerungseinrichtung nach einem der Ansprüche 16 bis 18, dadurch gekennzeichnet, daß die Empfangseinrichtung (41) ein Flächen-Array für Reflexion umfaßt.

23. Zertrümmerungseinrichtung nach einem der Ansprüche 1 bis 22, dadurch gekennzeichnet, daß eine Schalteinrichtung (54) zum Ein- und Abschalten einzelner Stoßwellenrohre vorgesehen ist.

**Claims**

1. Device for contactless fragmentation of a concretion located in the body of a living being using a shock-wave generator orientated towards a target area, characterised in that as shock wave generators several shock wave tubes (3), known in themselves, generating essentially plane shock waves, are provided, orientated in such a way that the centre lines (7) of the shock waves emanating from them intersect in the target area (1) at a point of intersection (11 ; 65).

2. Fragmentation device according to claim 1, characterised in that a mechanical device (5, 71) is provided for orientating the shock waves towards the target area (1).

3. Fragmentation device according to claim 2, characterised in that the mechanical device comprises fine drives (5) which are assigned to individual shock wave tubes (3) and which in each case are provided for setting the distance between the relevant shock wave tube (3) and the target area (1) (figures 1 to 3).

4. Fragmentation device according to claim 2 or 3, characterised in that the mechanical device comprises a mounting arm support (71), which serves for setting the position of the point of intersection (11) of all centre axes (7) (figure 7).

5. Fragmentation device according to one of claims 1 to 4, characterised in that the shock wave tubes (3) are arranged in such a way that their central axes (7) intersect at a common point of intersection (11) and that the exit openings (12) of the shock wave tubes (3) are all at the same distance from this point of intersection (11).

6. Fragmentation device according to one of claims 1 to 5, characterised in that for temporally variable arrival at the target area (1) of the shock waves a device is provided which controls the shock wave tubes (3) electrically in such a way that a temporal transposition of the transmitted shock waves results.

7. Fragmentation device according to one of claims 1 to 6, characterised in that a control unit (53) is assigned to it, which is designed so that the amplitude and/or the pulse form of the individual shock waves of the shock wave tubes (3) is adjustable.

8. Fragmentation device according to one of claims 1 to 7, characterised in that an acoustic focusing device (61) based on the lens principle is provided which in each case focuses the shock waves of the individual shock wave tubes (3) onto the target area (1) (figures 2 and 3).

9. Device for contactless fragmentation of a concretion located in the body of a living being using a shock wave generator orientated towards a target area, characterised in that as shock wave generators several shock wave tubes (3), known in themselves, generating essentially plane shock waves are provided, which are aligned in parallel and have a common acoustic focusing device (66) based on the lens principle, which focuses the shock waves of the individual shock wave generators (3) onto the target area (1) (figure 6).

10. Fragmentation device according to claim 8 or 9, characterised in that the focusing device comprises an acoustic converging lens (61 ; 62).

11. Fragmentation device according to one of claims 1 to 10, characterised in that the shock wave tubes (3) are able to be coupled to the living being by means of a coupling medium (47).

12. Fragmentation device according to one of claims 1 to 11, characterised in that the shock wave tubes (3) are able to be coupled to the living being (19) by way of a first flexible plastic bag (49) filled with a coupling medium (47), the coupling medium (47) having acoustic properties similar to those of water.

13. Fragmentation device according to claim 12, characterised in that the first plastic bag (49) is equipped with folds (51), which enable a bellows-like movement of the bag wall.

14. Fragmentation device according one of claims 1 to 13, characterised in that the shock waves after passing through the target area (1) are able to be decoupled from the body by way of a second plastic bag filled with a coupling medium.

15. Fragmentation device according to claim 12, 13 or 14, characterised in that the first and second plastic bag (49) are united in a third plastic bag (67) which can be placed around the target area (1).

16. Fragmentation device according to one of claims 1 to 5, characterised in that, for the purpose of location of the concretion, there is associated with it a device (41) for receiving and for processing transmitted and/or reflected acoustic signals which form through interaction of a transmitted pressure pulse with the body of the living being (19) and/or with the coupling medium.

17. Fragmentation device according to claim 16, characterised in that associated with it and integrated into the reception and processing device (41) is a device (41) for transmitting the pressure pulse lying in the ultra-sonic region.

18. Fragmentation device according to claim 16, characterised in that as transmitter for the pressure pulse lying in the ultra-sonic region at least one of the shock wave tubes (3) is provided.

19. Fragmentation device according to one of claims 16 to 18, characterised in that the device for receiving comprises an ultra-sonic array, which is rotatably adjustable along its longitudinal axis.

20. Fragmentation device according to one claims 16 to 19, characterised in that the receiving device (41) is arranged between the shock wave tubes (3) orientated towards the target area (1).

21. Fragmentation device according to one of claims 16 to 18, characterised in that the device (41) for receiving comprises a surface-array for transmission.

22. Fragmentation device according to one of claims 16 to 18, characterised in that the receiving device (41) comprises a surface-array for reflection.

23. Fragmentation device according to one of claims 1 to 22, characterised in that a switch device (54) is provided for switching on and off the individual shock wave tubes.

## Revendications

1. Dispositif pour détruire, sans contact, une concrétion située dans le corps d'un être vivant, à l'aide d'un générateur d'ondes de choc, qui est dirigé vers une région-cible, caractérisé par le fait qu'il est prévu, comme générateur d'ondes de choc, plusieurs tubes à ondes de choc (3), connus en soi, qui produisent des ondes de choc sensiblement planes et sont dirigés de telle sorte que les axes médians (7) des ondes de choc partant de ces tubes, se recoupent en un point d'intersection (11 ; 65) dans la région cible (1).

2. Dispositif de destruction selon la revendication 1, caractérisé en ce qu'il est prévu un dispositif mécanique (5, 71) servant à diriger les ondes de choc sur la région-cible (1).

3. Dispositif de destruction suivant la revendication 2, caractérisé par le fait que le dispositif mécanique comporte des dispositifs d'entraînement micrométriques (5), qui sont associés à des tubes individuels à ondes de choc (3) et sont prévus respectivement pour régler la distance entre le tube à ondes de choc (3) considéré et l'appareil de visée (1) (figures 1 à 3).

4. Dispositif de destruction suivant la revendication 2 ou 3, caractérisé par le fait que le dispositif mécanique comporte une structure (71) formant bras de maintien, qui sert à régler la position du point d'intersection (11) de tous les axes médians (7) (figure 7).

5. Dispositif de destruction suivant l'une des revendications 1 à 4, caractérisé par le fait que les tubes à ondes de choc (3) sont disposés de telle sorte que leurs axes centraux (7) se recoupent en un point d'intersection commun (11) et que les ouvertures de sortie (12) des tubes à ondes de choc (3) sont toutes situées à la même distance de ce point d'intersection (11).

6. Dispositif de destruction suivant l'une des revendications 1 à 5, caractérisé par le fait que pour obtenir une arrivée, modifiable dans le temps, des ondes de choc dans la région-cible (1) il est prévu un dispositif qui commande électriquement les tubes à ondes de choc (3) de manière à provoquer un décalage dans le temps des ondes de choc émises.

7. Dispositif de destruction suivant l'une des revendications 1 à 6, caractérisé par le fait qu'à ce dispositif est associée une unité de commande (53) qui est agencée de manière à permettre le réglage de l'amplitude et/ou de la forme des impulsions des différentes ondes de choc des tubes à ondes de choc (30).

8. Dispositif de destruction suivant l'une des revendications 1 à 7, caractérisé par le fait qu'il est prévu un dispositif de focalisation acoustique (61), qui est basé sur le principe des lentilles et focalise les ondes de choc des différents tubes à ondes de choc (3) respectivement sur la région-cible (1) (figures 2 et 3).

9. Dispositif pour détruire sans contact une concrétion située dans le corps d'un être vivant, comportant un générateur d'ondes de choc, qui est dirigé sur une région-cible, caractérisé par le fait qu'on utilise, comme générateur d'ondes de choc, plusieurs tubes à ondes de choc (3), connus en soi, qui produisent des ondes de choc sensiblement planes, sont orientés parallèlement entre eux et comportent un dispositif commun de focalisation acoustique (66) basé sur le principe des lentilles et focalisant les ondes de choc des différents générateurs d'ondes de choc (3) sur la région-cible (1) (figure 6).

10. Dispositif de destruction suivant la revendication 8 ou 9, caractérisé par le fait que le dispositif de focalisation comporte une lentille acoustique convergente (61 ; 62).

11. Dispositif de destruction suivant l'une des revendications 1 à 10, caractérisé par le fait que les tubes à ondes de choc (3) peuvent être accouplés à l'être vivant (19) au moyen d'un milieu d'accouplement (47).

12. Dispositif de destruction suivant l'une des revendications 1 à 11, caractérisé par le fait que les tubes à ondes de choc (3) peuvent être accouplés à l'être vivant (19) par l'intermédiaire d'un premier sac flexible en matière plastique (49) rempli par un milieu d'accouplement (47), dont les propriétés acoustiques sont semblables à celles de l'eau.

13. Dispositif de destruction suivant la revendication 12, caractérisé par le fait que le premier sac en matière plastique (49) comporte des replis (51), qui permettent un déplacement de la paroi du sac à la manière d'un soufflet.

14. Dispositif de destruction suivant l'une des revendications 1 à 13, caractérisé par le fait qu'après avoir traversé la région cible (1), les ondes de choc peuvent sortir du corps en étant découplées, par l'intermédiaire d'un second sac en matière plastique rempli par un milieu de couplage.

15. Dispositif de destruction suivant la revendication 12, 13 ou 14, caractérisé par le fait que les premier et second sacs en matière plastique (49) sont réunis pour former un troisième sac en matière plastique (67) pouvant être disposé autour de la région-cible (1).

16. Dispositif de destruction suivant l'une des revendications 1 à 5, caractérisé par le fait qu'à ce dispositif se trouve associé, en vue de la localisation de la concrétion, un dispositif (41) servant à recevoir et traiter des signaux acoustiques transmis et/ou réfléchis, qui apparaissent par suite de l'interaction d'une impulsion de pression émise avec le corps de l'être vivant (19) et/ou avec le milieu de couplage.

17. Dispositif de destruction suivant la revendication 16, caractérisé par le fait qu'à ce dispositif se trouve associé un dispositif (41) qui sert à émettre une impulsion de pression située dans la plage des ultrasons et est intégré dans le dispositif de réception et de traitement (41).

18. Dispositif de destruction suivant la revendication 16, caractérisé par le fait qu'on prévoit comme émetteur pour l'impulsion de pression située dans la plage des ultrasons, au moins l'un des tubes à ondes de choc (3).

19. Dispositif de destruction suivant l'une des revendications 16 à 18, caractérisé par le fait que le dispositif de réception comporte un réseau de réception des ultrasons, qui peut être déplacé en rotation autour de son axe longitudinal.

20. Dispositif de destruction suivant l'une des revendications 16 à 19, caractérisé par le fait que le dispositif de réception (41) est disposé entre les tubes à ondes de choc (3) dirigés sur la région-cible (1).

21. Dispositif de destruction suivant l'une des revendications 16 à 18, caractérisé par le fait que le dispositif de réception (41) comporte un ensemble de surfaces permettant de réaliser une transmission.

22. Dispositif de destruction suivant l'une des revendications 16 à 18, caractérisé par le fait que le dispositif de réception (41) comporte un ensemble de surfaces permettant une réflexion.

23. Dispositif de destruction suivant l'une des revendications 1 à 22, caractérisé par le fait qu'il est prévu un dispositif de commutation (54) servant à brancher et débrancher individuellement des tubes à ondes de choc.

FIG 1

FIG 2

**0 133 946**

FIG 3

FIG 4

FIG 5

FIG 6

FIG 7